(19) Europäisches Patentamt
European Patent Office
Office européen des brevets

(11) Numéro de publication : **0 073 161**
**B1**

(12) **FASCICULE DE BREVET EUROPÉEN**

(45) Date de publication du fascicule du brevet : **22.10.86**

(51) Int. Cl.[4] : **C 07 D237/20**, C 07 D237/34

(21) Numéro de dépôt : **82401426.0**

(22) Date de dépôt : **30.07.82**

(54) **Nouveaux dérivés de la pyrazine actifs sur le système nerveux central.**

(30) Priorité : **11.08.81 FR 8115546**

(43) Date de publication de la demande : **02.03.83 Bulletin 83/09**

(45) Mention de la délivrance du brevet : **22.10.86 Bulletin 86/43**

(84) Etats contractants désignés : **AT BE CH DE FR GB IT LI LU NL SE**

(56) Documents cités :
**FR-A- 2 141 697**
**EUR. J. MED. CHEM. - CHIMICA THERAPEUTICA, vol.11, no.2, mars-avril 1976 G. LECLERC et al.: "Synthèses et corrélations de Hansch dans une série d'hydrazinopyridazines hypotensives", pages 107 à 113**

(73) Titulaire : **SANOFI, société anonyme**
**40, Avenue George V**
**F-75008 Paris (FR)**

(72) Inventeur : **Chambon, Jean-Pierre**
**Chemin des Truquets Route de Montpellier**
**F-34570 Montarnaud (FR)**
Inventeur : **Wermuth, Camille**
**3 rue de la Côte d'Azur**
**F-67100 Strasbourg (FR)**

(74) Mandataire : **Gillard, Marie-Louise et al**
**Cabinet Beau de Loménie 55, Rue d'Amsterdam**
**F-75008 Paris (FR)**

**EP 0 073 161 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

Depuis de nombreuses années, des dérivés de pyridazine ont été proposés en tant que médicaments. Dans un grand nombre de cas, il s'agit de substances actives sur le système cardiovasculaire et présentant en particulier un effet hypotenseur ou vasodilatateur. On peut citer, par exemple, l'article du Eur. J. Med. Chem. Chemica Therapeutica *11* (1976), pages 107-113, où il est décrit des hydrazinopyridazines, notamment la méthylamino-3 méthyl-4 phényl-6 pyridazine, hypotensives.

Plus rarement, on a mentionné parmi les dérivés de la pyridazine une action anti-inflammatoire et analgésique. Enfin, le FR-A-2 141 697 décrit une famille de produits répondant à la formule générale :

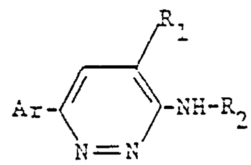

où :

$R_1$ représente l'hydrogène ou un groupe alkyle inférieur ;

Ar représente un reste aromatique ;

$R_2$ désigne un groupe

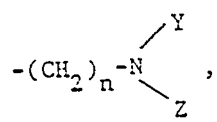

dans lequel n = 2 ou 3 et Y et Z représentent un groupe alkyle inférieur ou bien

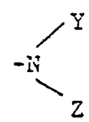

constitue un radical hétérocyclique.

Ces composés sont caractérisés par une action psychotrope de type psychostimulante.

Une étude ultérieure du composé où $R_1 = CH_3$, Ar = phényle et

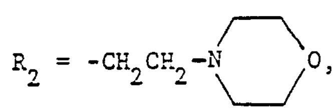

qui a reçu la Dénomination Commune Internationale « Minaprine », a permis de montrer qu'il s'agit d'une action psychotrope d'un type nouveau que l'on a désigné par activité « désinhibitrice ». Par ailleurs, à dose supérieure à 100 mg/kg *per os*, ce produit se montre convulsivant.

Il a été découvert aujourd'hui que, en modifiant la nature du substituant $R_2$, on peut de façon surprenante modifier fortement l'activité des composés obtenus qui deviennent alors anticonvulsivants.

La présente invention a pour objet une famille de dérivés de la pyridazine répondant à la formule générale (I) :

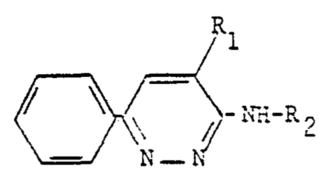

(I)

dans laquelle :

$R_1$ représente un groupe alkyle inférieur et notamment le groupe méthyle

$R_2$ représente un groupe

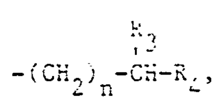

dans lequel n = 1, 2 ou 3, $R_3$ représente l'hydrogène ou un groupe méthyle et $R_4$ représente l'hydrogène, un groupe hydroxyle, un groupe éther $OR_5$, un groupe ester $OCOR_5$ dans lequel $R_5$ représente un groupe alkyle inférieur (1 à 4 carbones).

La présente invention concerne également les sels d'addition d'acides des composés de formule (I). Elle comprend aussi un procédé de préparation des composés de formule (I) ainsi que les compositions pharmaceutiques les contenant.

Elle concerne également l'(hydroxy-2 éthylamino)-3 diphényl-4,6 pyridazine, ses sels d'addition avec les acides, et les compositions pharmaceutiques les contenant. Lesdits produits sont obtenus par le même procédé de préparation que celui décrit ci-dessous pour les composés de formule (I).

Les composés (I) sont obtenus à partir d'une chloro-3 pyridazine convenablement substituée (II) selon le schéma réactionnel :

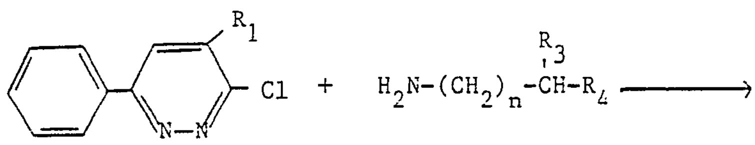

(I)

(II) (III)

La réaction entre le dérivé chloré (II) et l'amine (III) s'effectue en général par chauffage au sein d'un solvant convenable, tel qu'un alcool, le plus souvent à température d'ébullition du solvant. La durée de la réaction varie de quelques heures à plusieurs jours suivant la nature des réactifs mis en jeu. Lorsque la réaction s'avère trop lente, elle peut être catalysée par addition d'une petite quantité de poudre de cuivre.

On effectue la réaction en présence d'un accepteur d'hydracide destiné à fixer l'acide chlorhydrique formé dans la réaction. Le plus souvent, on utilise comme tel un excès de l'amine (III).

L'isolement du composé (I) s'effectue par reprise dans l'eau et extraction par un solvant convenable, tel que l'acétate d'éthyle.

Les composés (I) ainsi obtenus peuvent être salifiés de façon classique par action de l'acide sur une solution chaude de la base, le solvant étant choisi de façon que le sel cristallise par refroidissement.

Lorsque $R_4$ représente un groupe $OCOR_5$, une variante du procédé consiste à préparer le composé (I) correspondant où $R_4$ représente OH puis à acyler ce dernier selon un procédé habituel, par exemple par action du chlorure d'acide $R_5COCl$ au sein de la pyridine.

Les exemples suivants sont donnés à titre d'illustration de la présente invention.

## Exemple 1

Butylamino-3 méthyl-4 phényl-6 pyridazine.
(Tartrate) (CM 30 434)

$$R_1 = CH_3 \; ; \qquad (I)$$

$$R_2 = -(CH_2)_3CH_3$$

On porte au reflux pendant 48 heures le mélange de 10,3 g de chloro-3 méthyl-4 phényl-6 pyridazine et 7,5 g de butylamine dans 100 ml de butanol. On verse le mélange dans 200 ml d'eau et extrait avec de l'acétate d'éthyle. On sépare la phase organique et on l'extrait avec une solution aqueuse d'acide sulfurique 5N. L'extrait acide est alcalinisé par addition de bicarbonate de sodium puis on extrait avec du chloroforme. On lave la solution avec de l'eau, sèche et évapore le solvant. On obtient un produit huileux.

On dissout cette huile dans l'isopropanol chaud et ajoute un équivalent d'acide tartrique et chauffe jusqu'à dissolution. Après refroidissement, on essore un solide incolore qu'on recristallise dans l'isopropanol ; F. 184-186 °C ; rendement 12,5 g.

Le tartrate cristallise avec 2 molécules d'eau.

En opérant de la même façon, mais en remplaçant la butylamine par une quantité équivalente d'octylamine, on obtient le tartrate d'octylamino-3 méthyl-4 phényl-6 pyridazine (CM 30 435) ; F. 164-166 °C (isopropanol-éther) ; rendement 54 %.

## Exemple 2

(Hydroxy-2 éthylamino)-3 méthyl-4 phényl-6 pyridazine.
(Chlorhydrate) (CM 30 094)

$$R_1 = CH_3 \; ; \qquad (I)$$

$R_2 = —CH_2CH_2OH$

On porte au reflux pendant 3 jours le mélange de 30,6 g de chloro-3 méthyl-4 phényl-6 pyridazine, 36,6 g d'amino-2 éthanol et 0,1 g de poudre de cuivre dans 400 ml de butanol.

On verse le mélange dans 500 ml d'eau et filtre la solution sur Büchner. On extrait avec de l'acétate d'éthyle et sèche la solution sur sulfate de magnésium. On évapore le solvant à siccité. Le résidu cristallise. On recristallise dans un mélange acétate d'éthyle-isopropanol (2-1 v/v). On obtient des cristaux incolores (20 g) : F. 151 °C.

Chlorhydrate :

On dissout 11,45 g de base dans 100 ml d'isopropanol en chauffant jusqu'à dissolution. On ajoute 4,7 ml d'acide chlorhydrique concentré et laisse cristalliser par refroidissement. On recristallise deux fois dans le méthanol.

On obtient un solide incolore (7 g) ; F. 200 °C.

Exemples 3 à 6

En faisant varier l'aminoalcool ou le dérivé chloré utilisés, on obtient les composés (I) figurant dans le tableau (I).

Tableau I

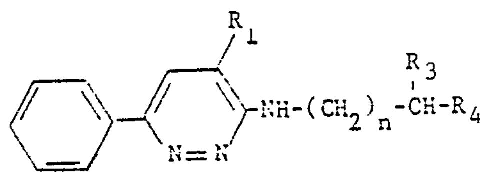

| N° de code - CM | $R_1$ | n | $R_3$ | $R_4$ | Durée de la réaction en heures | Base F., °C (solvant) | Chlorhydrate F., °C (solvant) |
|---|---|---|---|---|---|---|---|
| 30 095 | $-CH_3$ | 2 | H | OH | 72 | 134 (isopropanol) | 209 (éthanol à 95) |
| 30 096 | $-CH_3$ | 1 | $CH_3$ | OH | 72 | 114 (acétate d'éthyle) | 193 (éthanol à 95) |
| 30 097 | $-CH_3$ | 3 | H | OH | 72 | 93 (acétate d'éthyle) | 196 (éthanol à 95) |
| 30 339 | 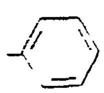 | 1 | H | OH | 48 | solide hygroscopique | 182 (isopropanol) |

Exemple 7

(Propionyloxy-2 éthylamino)-3 méthyl-4 phényl-6 pyridazine.
(Malate) (CM 30 098)

$R_1 = CH_3$ ; (I)

$R_2 = CH_2CH_2OCOCH_2CH_3$

On dissout 16 g du composé CM 30 094, base obtenue à l'exemple 2, dans 250 ml de pyridine par agitation à température ambiante. On ajoute ensuite goutte à goutte, en poursuivant l'agitation, 6,3 ml de chlorure de propionyle. Après la fin de l'addition, on continue à agiter pendant 2 heures à température ambiante puis on évapore à siccité. Le résidu dissous dans le minimum de chloroforme est chromatographié sur une colonne de silice. En éluant avec un mélange acétate d'éthyle-hexane (50-50 v/v), on obtient après évaporation du solvant un solide incolore.

Après recristallisation dans un petit volume d'acétate d'éthyle : F. 102 °C ; poids 8 g.

Malate :

On dissout 8 g de base dans 100 ml d'isopropanol chaud puis on ajoute la solution chaude de 4,02 g d'acide malique dans 10 ml d'isopropanol. Après refroidissement et addition d'un peu d'éther anhydride, on isole un solide incolore qu'on recristallise deux fois dans l'acétonitrile.

F. 110 °C, poids 6,5 g

De la même façon, en remplaçant le chlorure de propionyle par une quantité équivalente de chlorure d'acétyle, on isole par le même traitement l'(acétoxy-2 éthylamino)-3 méthyl-4 phényl-6 pyridazine ; F. 127 °C.

Malate ; F. 102-105 °C (acétonitrile).

## Exemple 8

(Méthoxy-2 éthylamino)-3 méthyl-4 phényl-6 pyridazine.
(Chlorhydrate) (CM 30 310)

$$R_1 = CH_3 ; \qquad (I)$$

$$R_2 = -CH_2-CH_2-O-CH_3$$

On porte au reflux pendant 4 jours un mélange de 7 g de chloro-3 méthyl-4 phényl-6 pyridazine et 7 g de méthoxy-2 éthylamine dans 50 ml de butanol. On verse la solution chaude dans 200 ml d'eau et extrait avec de l'acétate d'éthyle. On sèche la solution organique sur sulfate de magnésium puis évapore à siccité.

On distille le résidu sous vide poussé ; E. /0,1 mm ; F. 208-210 °C ; poids 6,8 g.

Chlorhydrate :

On dissout 6,8 g de la base dans l'isopropanol chaud puis on ajoute 2,5 ml d'acide chlorhydrique concentré et laisse cristalliser ; F. 194 °C ; poids 6,5 g.

Cristallise avec 1/2 molécule d'eau.

En opérant de même à partir de la chloro-3 diphényl-4,6 pyridazine, on obtient de la même façon la (méthoxy-2 éthylamino)-3 diphényl-4,6 pyridazine (CM 30 340).

Base ; F. 92 °C (éther isopropylique).

Chlorhydrate ; F. 193 °C (isopropanol).

Les produits selon l'invention ont été étudiés en ce qui concerne leur activité pharmacologique.

1. Activité anticonvulsivante

L'activité anticonvulsivante des dérivés a été évaluée vis-à-vis de deux agents chimiques : strychnine et bicuculline et vis-à-vis de l'électrochoc. Dans ces différents essais, les produits ont été comparés à la minaprine décrite dans le brevet français n° 2 141 697 cité ci-dessus.

Les produits ont également été comparés au sel de sodium de l'acide valproïque (DCI) dont l'activité anticonvulsivante est bien connue et utilisée en thérapeutique humaine.

Activité antistrychnine :

Les produits sont administrés par voie orale 30 min avant la strychnine 0,9 mg/kg i.p. L'apparition des crises tétaniques ainsi que la mortalité sont relevées pendant les 60 min qui suivent l'administration de la strychnine.

Activité antibicuculline :

Les produits sont administrés par voie orale 30 min avant la bicuculline 0,9 mg/kg i.v. L'apparition des crises cloniques, toniques ainsi que la mortalité sont relevées pendant les 60 min qui suivent l'administration de bicuculline.

Activité antiélectrochoc :

Les produits ont été administrés *per os* 30 min avant l'électrochoc (12,5 V pendant 0,5 s). Le choc électrique a été délivré à l'animal par l'intermédiaire d'électrodes cornéennes. L'apparition des convulsions clinico-toniques chez les animaux témoins a été immédiate. Les animaux ne présentant pas l'extension des membres postérieurs ont été considérés comme protégés.

Dans les trois cas, on administre les produits en gamme de doses à des lots de 10 souris par dose.

Pour chaque produit, on détermine la dose efficace 50 ($DE_{50}$) ou dose qui antagonise l'effet convulsivant de l'agent utilisé chez 50 % des animaux traités.

Dans le tableau III ci-après sont réunis les résultats obtenus avec divers produits représentatifs de l'invention.

Les produits de l'invention manifestent une activité anticonvulsivante importante. Vis-à-vis de la strychnine, cette activité est particulièrement forte pour les composés CM 30 096, 30 310 et 30 339. Vis-à-vis de la bicuculline et de l'électrochoc, l'activité anticonvulsivante est un peu moins puissante mais est toutefois notable, en particulier pour les dérivés CM 30 096 et 30 310.

En ce qui concerne les produits de référence, la minaprine est totalement inactive dans ces tests jusqu'à la dose maximale tolérée. Quant au valproate de sodium administré par voie orale, il présente dans les mêmes conditions expérimentales une dose efficace 50 le plus souvent supérieure à celle des produits de l'invention.

Tableau II

| Produits | Inhibition des effets convulsivants de | | |
|---|---|---|---|
| | la strychnine $DE_{50}$ (mg/kg) | la bicuculline $DE_{50}$ (mg/kg) | l'électrochoc $DE_{50}$ (mg/kg) |
| CM 30 094 | 119 | 286 | 107 |
| CM 30 096 | 92 | 175 | 209 |
| CM 30 310 | 158 | 136 | 78 |
| CM 30 339 | 145 | 86 | ∼ 200 |
| Minaprine | inactif | inactif | inactif |
| Valproate de sodium | 480 | 234 | 290 |

2. Toxicité aiguë

Les produits à étudier ont été administrés par voie orale aux doses de 250, 500 et 1 000 mg/kg à des lots de 5 souris. La mortalité provoquée par les dérivés a été estimée pendant les 24 heures qui ont suivi l'administration du produit.

Tableau III

| N° produit | % de mortalité | | |
|---|---|---|---|
| | à 1000 mg/kg | à 500 mg/kg | à 250 mg/kg |
| Minaprine | 100 | 100 | 100 |
| CM 30 094 | 0 | 0 | 0 |
| CM 30 096 | 100 | 20 | 0 |
| CM 30 097 | 100 | 0 | 0 |
| CM 30 310 | 100 | 0 | 0 |
| CM 30 339 | 60 | 60 | 0 |
| CM 30 340 | 20 | 0 | 0 |
| CM 30 434 | 0 | 0 | 0 |
| CM 30 451 | 0 | 0 | 0 |
| CM 30 462 | 40 | 20 | 0 |

Les résultats exprimés en pourcentage d'animaux qui meurent après administration de divers produits de l'invention sont notés dans le tableau précédent. Tous les produits étudiés sont dénués de toxicité à la dose de 250 mg/kg *per os.* A la dose de 500 mg/kg *per os,* seuls les dérivés CM 30 096, CM 30 339 et CM 30 462 ont un effet toxique. La minaprine possède un effet toxique beaucoup plus important, elle provoque 20 % de mortalité dès la dose de 100 mg/kg *per os* chez la souris.

Les essais ainsi effectués montrent que les produits selon l'invention présentent des propriétés pharmacologiques intéressantes et une faible toxicité. Par suite, ils peuvent être utilisés en thérapeutique humaine, notamment pour le traitement des affections psychiques, neurologiques ou neuromusculaires.

En particulier, les produits selon l'invention peuvent être utilisés pour le traitement des troubles de l'humeur ou du comportement : nervosisme, irritabilité ainsi que pour les traitements des états anxieux et des insomnies.

Ces produits peuvent être administrés par voie orale ou par voie injectable. Les compositions pharmaceutiques peuvent être solides ou liquides et se présenter par exemple sous forme de comprimés, gélules, granulés, suppositoires ou préparations injectables.

La posologie peut varier dans de larges proportions, en particulier suivant le type et la gravité de l'affection à traiter et suivant le mode d'administration. Le plus souvent chez l'adulte par voie orale, elle est comprise entre 0,050 et 0,500 g par jour, éventuellement répartie en plusieurs prises.

A titre d'exemple, on peut indiquer les préparations galéniques suivantes :

Comprimés

| | |
|---|---|
| CM 30 310 | 200 mg |
| Cellulose microcristalline | 100 mg |
| Lactose | 197 mg |
| Stéarate de magnésium | 3 mg |
| | 500 mg |

**Revendications** (pour les Etats contractants : BE, CH, DE, FR, GB, IT, LI, LU, NL, SE)

1. Dérivés de la pyridazine de formule générale (I) :

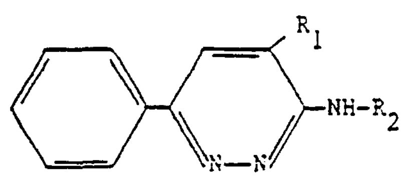

(I)

dans laquelle .

$R_1$ est un groupe alkyle ayant de 1 à 4 atomes de carbone,

$R_2$ représente un groupe

$$-(CH_2)_n-\overset{R_3}{\overset{|}{C}H}-R_4$$

dans lequel n = 1, 2 ou 3,

$R_3$ représente l'hydrogène ou un groupe méthyle et

$R_4$ est choisi parmi l'hydrogène, un groupe hydroxy, un groupe éther $OR_5$ ou un groupe ester $OCOR_5$ dans lesquels $R_5$ représente un radical alkyle ayant de 1 à 4 atomes de carbone,

ainsi que les sels d'addition desdits dérivés avec les acides.

2. (Hydroxy-2 éthylamino)-3 diphényl-4,6 pyridazine ou l'un de ses sels d'addition avec les acides.

3. Dérivés selon la revendication 1, caractérisés en ce que $R_1$ est $CH_3$ et $R_4$ est OH.

4. Procédé de préparation des produits de formule (I) selon la revendication 1 caractérisé en ce que l'on utilise comme produit de départ une chloro-3 pyridazine de formule (II) :

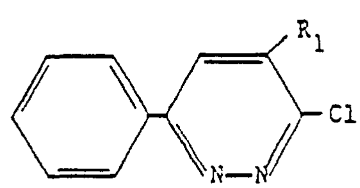

(II)

dans laquelle $R_1$ est tel que défini à la revendication 1, que l'on fait réagir avec une amine de formule (III) :

$$H_2N-(CH_2)_n-\overset{\overset{R_3}{|}}{C}H-R_4 \qquad \text{(III)}$$

dans laquelle n, $R_3$ et $R_4$ sont tels que définis à la revendication 1, ladite réaction étant réalisée au sein d'un solvant tel qu'un alcool, de préférence à la température d'ébullition dudit solvant et en présence d'un accepteur d'hydracide ; en ce que, éventuellement on traite le composé obtenu de formule (I) dans laquelle $R_4 = OH$ par un chlorure d'acide de formule $R_5COCl$ dans laquelle $R_5$ est tel que défini à la revendication 1, en présence de pyridine, et en ce qu'éventuellement on salifie les composés de formule (I) obtenu.

5. Procédé de préparation des produits selon la revendication 2, caractérisé en ce que l'on utilise comme produit de départ la chloro-3 diphényl-4,6 pyridazine, que l'on fait réagir avec l'amino-2 éthanol-1, ladite réaction étant réalisée au sein d'un solvant tel qu'un alcool, de préférence à la température d'ébullition dudit solvant et en présence d'un accepteur d'hydracide et en ce qu'éventuellement on salifie le produit ainsi obtenu.

6. Procédé selon l'une des revendications 4 ou 5, caractérisé en ce que la réaction est réalisée en présence de cuivre, utilisé comme catalyseur.

7. Compositions pharmaceutiques, utilisables notamment pour le traitement des affections psychiques, neurologiques ou neuromusculaires, caractérisées en ce qu'elles contiennent en association avec un excipient pharmaceutiquement acceptable, un produit selon l'une des revendications 1, 2 ou 3.

**Revendications** (pour l'Etat contractant AT)

1. Procédé pour la préparation de dérivés de la pyridazine de formule générale (I) :

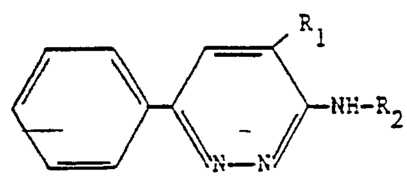

(I)

dans laquelle :

$R_1$ est un groupe alkyle ayant de 1 à 4 atomes de carbone,

$R_2$ représente un groupe

$$-(CH_2)_n-\overset{\overset{R_3}{|}}{C}H-R_4$$

dans lequel n = 1, 2 ou 3,

$R_3$ représente l'hydrogène ou un groupe méthyle et

$R_4$ est choisi parmi l'hydrogène, un groupe hydroxy, un groupe éther $OR_5$ ou un groupe ester $OCOR_5$ dans lesquels $R_5$ représente un radical alkyle ayant de 1 à 4 atomes de carbone,

ainsi que des sels d'addition desdits dérivés avec des acides, caractérisé en ce que l'on utilise comme produit de départ une chloro-3 pyridazine de formule (II) :

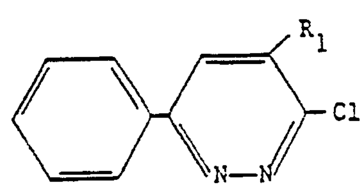

(II)

dans laquelle $R_1$ est tel que défini ci-dessus, que l'on fait réagir avec une amine de formule (III) :

$$H_2N-(CH_2)_n-\overset{\overset{R_3}{|}}{C}H-R_4 \qquad \text{(III)}$$

dans laquelle n, $R_3$ et $R_4$ sont tels que définis ci-dessus, ladite réaction étant réalisée au sein d'un solvant tel qu'un alcool, de préférence à la température d'ébullition dudit solvant et en présence d'un accepteur d'hydracide ; en ce qu'éventuellement on traite le composé obtenu de formule (I) dans laquelle $R_4$ = OH par un chlorure d'acide de formule $R_5COCl$, dans laquelle $R_5$ est tel que défini ci-dessus, en présence de pyridine et en ce qu'éventuellement on salifie les composés de formule (I) obtenus.

2. Procédé pour la préparation de l'(hydroxy-2 éthylamino)-3 diphényl-4,6 pyridazine ou de l'un de ses sels d'addition avec les acides caractérisé en ce que l'on utilise comme produit de départ la chloro-3 diphényl-4,6 pyridazine, que l'on fait réagir avec l'amino-2 éthanol-1, ladite réaction étant réalisée au sein d'un solvant tel qu'un alcool, de préférence à la température d'ébullition dudit solvant et en présence d'un accepteur d'hydracide et en ce qu'éventuellement on salifie le produit ainsi obtenu.

3. Procédé selon l'une des revendications 1 ou 2, caractérisé en ce que la réaction est réalisée en présence de cuivre, utilisé comme catalyseur.

4. Utilisation des dérivés de la pyridazine préparés selon l'une des revendications 1 ou 2, pour la préparation de compositions pharmaceutiques, utilisables notamment pour le traitement des affections psychiques, neurologiques ou neuromusculaires.

**Claims** (for the Contracting States : BE, CH, DE, FR, GB, IT, LI, LU, NL, SE)

1. Derivatives of pyridazine of general formula (I) :

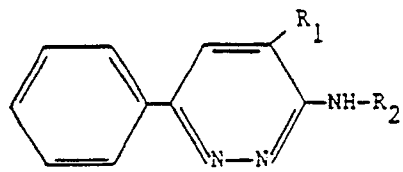

(I)

in which :

$R_1$ is an alkyl group having 1 to 4 carbon atoms,

$R_2$ is a

$$-(CH_2)_n-\overset{R_3}{\overset{|}{C}}H-R_4$$

group, in which n = 1, 2 or 3,

$R_3$ is hydrogen or a methyl group, and

$R_4$ is selected from hydrogen, a hydroxy group, a $OR_5$ ether group or a $OCOR_5$ ester group in which $R_5$ is an alkyl radical having 1 to 4 carbon atoms ;

as well as the addition salts of said derivatives with the acids.

2. 3-(2-hydroxy-ethylamino)-4,6-diphenyl-pyridazine or one of its addition salts with the acids.

3. Derivatives according to claim 1, characterized in that $R_1$ is $CH_3$ and $R_4$ is OH.

4. Process for preparing the products of formula (I) according to claim 1, characterized in that it comprises the step of using, as starting product, a 3-chloro-pyridazine of formula (II)

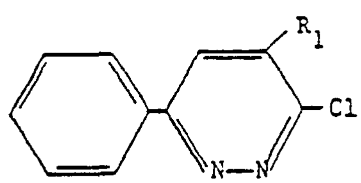

(II)

in which $R_1$ is such as defined above in claim 1 which product is reacted with an amine of formula (III) :

$$H_2N-(CH_2)_n-\overset{R_3}{\overset{|}{C}}H-R_4 \qquad \text{(III)}$$

in which n, $R_3$ and $R_4$ are such as defined in claim 1, said reaction being carried out in a solvent such as an alcohol, preferably at the boiling temperature of said solvent and in the presence of a hydracid acceptor ; in that the resulting compound of formula (I) in which $R_4$ = OH is optionally treated by an acid chloride of formula $R_5COCl$ in which $R_5$ is such as defined in claim 1, in the presence of pyridine, and in that the resulting compounds of formula (I) are optionally salified.

5. Process for preparing the compounds according to claim 2, characterized in that the 3-chloro-4,6-diphenyl pyridazine is used as starting product, and is reacted with the 2-amino-1-ethanol, said reaction being carried out in a solvent such as an alcohol, preferably at the boiling temperature of said solvent and in the presence of a hydracid acceptor, and in that the resulting product is optionally salified.

6. Process according to any one of claims 4 or 5, characterized in that the reaction is carried out in the presence of copper, used as a catalyst.

7. Pharmaceutical compositions usable in particular for the treatment of psychic, neurological or neuromuscular disorders, characterized in that they contain in association with a pharmaceutically acceptable excipient a product according to any one of claims 1, 2 or 3.

**Claims** (for the Contracting State AT)

1. Process for preparing pyridazine derivatives of general formula (I) :

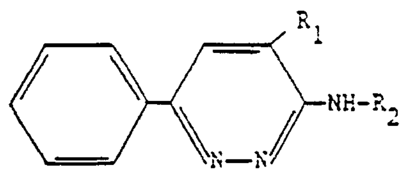

(I)

in which :

$R_1$ is an alkyl group having 1 to 4 carbon atoms,

$R_2$ is a

$$-(CH_2)_n-\underset{}{CH}(R_3)-R_4$$

group, in which n = 1, 2 or 3,

$R_3$ is hydrogen or a methyl group, and

$R_4$ is selected from hydrogen, a hydroxy group, a $OR_5$ ether group, a $OCOR_5$ ester group in which $R_5$ is an alkyl radical having 1 to 4 carbon atoms,

as well as the addition salts of said derivatives with the acids, characterized in that it comprises the step of using as starting product, a 3-chloro pyridazine of formula (II)

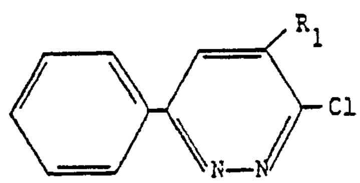

(II)

in which $R_1$ is such as defined above, which product is reacted with an amine of formula (III) :

$$H_2N-(CH_2)_n-CH(R_3)-R_4 \qquad (III)$$

in which n, $R_3$ and $R_4$ are such as defined above, said reaction being carried out in a solvent such as an alcohol, preferably at the boiling temperature of said solvent and in the presence of a hydracid acceptor ; in that the resulting compound of formula (I) in which $R_4$ = OH, is optionally treated by an acid chloride of formula $R_5COCl$ in which $R_5$ is such as defined above, in the presence of pyridine, and in that the resulting compounds of formula (I) are optionally salified.

2. Process for preparing the 3-(2-hydroxyethylamino)-4,6-diphenyl-pyridazine or one of its addition salts with the acids, characterized in that the 3-chloro-4,6-diphenyl pyridazine is used as starting product, and is reacted with the 2-amino-1-ethanol, said reaction being carried out in a solvent such as an alcohol, preferably at the boiling temperature of said solvent and in the presence of a hydracid acceptor and in that the resulting product is optionally salified.

3. Process according to any one of claims 1 or 2, characterized in that the reaction is carried out in the presence of copper, used as a catalyst.

4. Use of pyridazine derivatives prepared according to any one of claims 1 or 2, for preparing pharmaceutical compositions usable in particular for the treatment of psychic, neurological or neuromuscular treatment.

**Patentansprüche** (für die Vertragsstaaten : BE, CH, DE, FR, GB, IT, LI, LU, NL, SE)

1. Pyridazinderivate der allgemeinen Formel (I) :

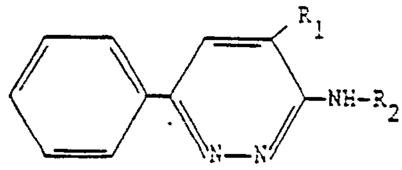

(I)

worin

$R_1$ eine Alkylgruppe mit 1 bis 4 Kohlenstoffatomen darstellt,

$R_2$ für eine Gruppe

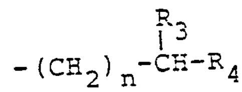

steht, worin n = 1, 2 oder 3,

- $R_3$ Wasserstoff oder eine Methylgruppe bedeutet und

$R_4$ ausgewählt ist aus Wasserstoff, einer Hydroxygruppe, einer Äthergruppe $OR_5$ oder einer Estergruppe $OCOR_5$, worin $R_5$ einen Alkylrest mit 1 bis 4 Kohlenstoffatomen bedeutet, sowie die Säureadditionssalze dieser Derivate.

2. 3-(2-Hydroxyäthylamino)-4,6-diphenyl-pyridazin oder eines seiner Säureadditionssalze.

3. Derivate nach Anspruch 1, dadurch gekennzeichnet, daß $R_1$ für $CH_3$ steht und $R_4$ für OH steht.

4. Verfahren zur Herstellung der Produkte der Formel (I) nach Anspruch 1, dadurch gekennzeichnet, daß man als Ausgangsprodukt 3-Chlor-pyridazin der Formel (II)

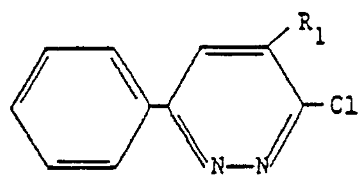

(II)

worin $R_1$ die in Anspruch 1 angegebene Bedeutung hat, einsetzt, welches man mit einem Amin der Formel (III)

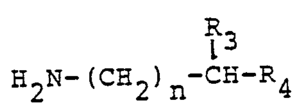

(III)

worin n, $R_3$ und $R_4$ die in Anspruch 1 angegebene Bedeutung haben, reagieren läßt, wobei die Reaktion in einem Lösungsmittel, wie einem Alkohol, vorzugsweise bei Kochtemperatur des Lösungsmittels und in Gegenwart eines Wasserstoffsäureakzeptors durchgeführt wird ; daß man eine erhaltene Verbindung der Formel (I), worin $R_4$ = OH, gegebenenfalls, mit einem Säurechlorid der Formel $R_5COCl$, worin $R_5$ die in Anspruch 1 angegebene Bedeutung hat, in Gegenwart von Pyridin behandelt, und daß man gegebenenfalls die erhaltenen Verbindungen der Formel (I) in ein Salz umwandelt.

5. Verfahren zur Herstellung der Produkte nach Anspruch 2, dadurch gekennzeichnet, daß man als Ausgangsprodukt 3-Chlor-4,6-diphenyl-pyridazin verwendet, welches man mit 2-Amino-äthanol-1 reagieren läßt, wobei die Reaktion in einem Lösungsmittel, wie einem Alkohol, vorzugsweise bei der Kochtemperatur des Lösungsmittels und in Gegenwart eines Wasserstoffsäureakzeptors durchgeführt wird, und daß man das so erhaltene Produkt gegebenenfalls in ein Salz überführt.

6. Verfahren nach einem der Ansprüche 4 oder 5, dadurch gekennzeichnet, daß die Reaktion in Gegenwart von Kupfer als Katalysator durchgeführt wird.

7. Pharmazeutische Zusammensetzungen, die insbesondere zur Behandlung von psychischen, neurologischen oder neuromuskulären Erkrankungen nützlich sind, dadurch gekennzeichnet, daß sie ein Produkt nach einem der Ansprüche 1, 2 oder 3 in Verbindung mit einem pharmazeutisch akzeptablen Exzipienten enthalten.

**Patentansprüche** (für den Vertragsstaat AT)

1. Verfahren zur Herstellung von Pyridazinderivaten der allgemeinen Formel (I) :

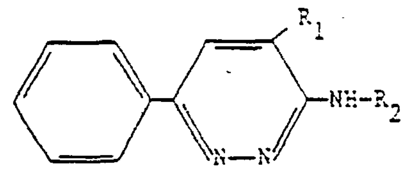

(I)

worin

$R_1$ eine Alkylgruppe mit 1 bis 4 Kohlenstoffatomen darstellt,

$R_2$ für eine Gruppe

$$-(CH_2)_n-\overset{\displaystyle R_3}{\overset{|}{C}}H-R_4$$

steht, worin n = 1, 2 oder 3,

$R_3$ Wasserstoff oder eine Methylgruppe bedeutet und

$R_4$ ausgewählt ist aus Wasserstoff, einer Hydroxygruppe, einer Äthergruppe $OR_5$ oder einer Estergruppe $OCOR_5$, worin $R_5$ einen Alkylrest mit 1 bis 4 Kohlenstoffatomen bedeutet,

sowie der Säureadditionssalze dieser Derivate, dadurch gekennzeichnet, daß man als Ausgangsprodukt 3-Chlor-pyridazin der Formel (II)

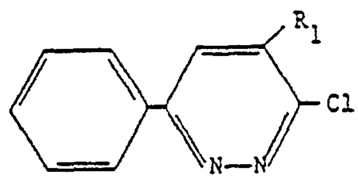

(II)

worin $R_1$ die oben angegebene Bedeutung hat, einsetzt, welches man mit einem Amin der Formel (III)

$$H_2N-(CH_2)_n-\overset{\displaystyle R_3}{\overset{|}{C}}H-R_4 \qquad \text{(III)}$$

worin n, $R_3$ und $R_4$ die in Anspruch 1 angegebene Bedeutung haben, reagieren läßt, wobei die Reaktion in einem Lösungsmittel, wie einem Alkohol, vorzugsweise bei Kochtemperatur des Lösungsmittels und in Gegenwart eines Wasserstoffsäureakzeptors durchgeführt wird ; daß man eine erhaltene Verbindung der Formel (I), worin $R_4$ = OH, gegebenenfalls mit einem Säurechlorid der Formel $R_5COCl$, worin $R_5$ die in Anspruch 1 angegebene Bedeutung hat, in Gegenwart von Pyridin behandelt, und daß man gegebenenfalls die erhaltenen Verbindungen der Formel (I) in ein Salz umwandelt.

2. Verfahren zur Herstellung von 3-(2-Hydroxy-äthylamino)-4,6-diphenyl-pyridazin oder eines seiner Säureadditionssalze, dadurch gekennzeichnet, daß man als Ausgangsprodukt 3-Chlor-4,6-diphenyl-pyridazin verwendet, welches man mit 2-Amino-äthanol-1 reagieren läßt, wobei die Reaktion in einem Lösungsmittel, wie einem Alkohol, vorzugsweise bei der Kochtemperatur des Lösungsmittels und in Gegenwart eines Wasserstoffsäureakzeptors durchgeführt wird, und daß man das so erhaltene Produkt gegebenenfalls in ein Salz überführt.

3. Verfahren nach einem der Ansprüche 1 oder 2, dadurch gekennzeichnet, daß die Reaktion in Gegenwart von Kupfer als Katalysator durchgeführt wird.

4. Verwendung der nach einem der Ansprüche 1 oder 2 hergestellten Pyridazinderivate zur Herstellung von pharmazeutischen Zusammensetzungen, die insbesondere zur Behandlung von psychischen, neurologischen oder neuromuskulären Erkrankungen nützlich sind.